# FASCICULE DE BREVET EUROPEEN

(11) **EP 0 443 926 B1**
(45) Date de publication et mention de la délivrance du brevet: **24.05.1995**
(21) Numéro de dépôt: 91400414.8
(22) Date de dépôt: 18.02.1991
(51) Int. Cl.: C12P 7/26

(54) **Procédé d'obtention d'irone par voie enzymatique**
Verfahren für die enzymatische Erlangung von Irone
Process for the enzymatic obtention of irone

(30) Priorité: 22.02.1990 FR 9002212
(43) Date de publication de la demande: 28.08.1991
(73) Titulaire: ELF AQUITAINE, 92400 Courbevoie (FR)
(72) Inventeur: Gil, Gérard, F-13400 Aubagne (FR); Le Petit, Jean, F-13190 Allauch (FR); Seris, Jean-Louis, F-64110 Jurançon (FR)
(74) Mandataire: Gillard, Marie-Louise

(56) Documents cités:
- FR-A- 2 620 702

## Description

La présente invention concerne un procédé d'obtention d'irone, dont les mélanges des trois isomères, α, β et γ, sont utilisés dans l'industrie alimentaire, celle des parfums et des cosmétiques, pour leur odeur de violette.

Lors de leur récolte, les rhizomes d'iris ne contiennent pratiquemment pas d'irone et c'est au cours d'un stockage prolongé que les molécules odorantes apparaissent, d'après Z. Naturforsch. 38c 179-184 (1983), vraisemblablement par dégradation oxydative de terpénoïdes.

Dans le procédé classique de préparation d'irone, cette oxydation s'effectue très lentement dans les rhizomes décortiqués, conservés dans des lieux frais et aérés, et on doit attendre plus de deux ans après la récolte pour extraire une quantité raisonnable d'irone des rhizomes par un solvant organique ou par entraînement à la vapeur.

Néanmoins, il a été montré récemment dans la demande FR-A-2 620 702 que l'oxydation chimique des précurseurs, en particulier triterpéniques, extraits des rhizomes par un solvant lipophile sans attendre leur maturation, permettait d'obtenir l'irone dans des conditions économiquement intéressantes. En effet, les rendements en irone extrait par rapport à l'unité de poids de rhizome sec sont supérieurs dans ce procédé qui, en outre, n'implique ni le décortiquage des rhizomes, nécessaire pour que la maturation à l'air s'effectue convenablement, ni leur conservation prolongée.

Le procédé selon l'invention implique aussi l'oxydation des précurseurs extraits des rhizomes dès leur récolte, mais sans que la durée écoulée soit critique et, après éventuellement leur séchage à l'air, pour éliminer une partie de l'eau et faciliter leur transport. Cette oxydation n'est plus effectuée par voie chimique, ce qui interdit l'utilisation de l'irone obtenue dans l'industrie alimentaire, mais par voie enzymatique en présence d'un système peroxydant. Ce procédé, dont la mise en oeuvre est particulièrement simple, permet d'obtenir l'irone avec des rendements d'au moins trois fois ceux du procédé classique.

Selon l'invention, on extrait les précurseurs des rhizomes d'iris frais, plus ou moins desséchés, par un solvant des molécules lipophiles, et on soumet le résidu d'extraction à l'action d'un système enzymatique peroxydant comprenant de l'eau oxygénée et une peroxydase, ou de l'oxygène et une lipoxygènase, éventuellement en présence d'un substrat de l'enzyme, par exemple l'acide dihydroxyfumarique ou l'acide ascorbique pour la peroxydase ou d'un acide carboxylique aliphatique insaturé, susceptible de donner en présence de la lipoxygènase un hydroperoxyde, comme l'acide linoléique, l'acide linolénique ou l'acide arachidonique.

La première étape d'extraction des précurseurs terpéniques des rhizomes est effectuée sur les rhizomes concassés ou même broyés, de préférence avec un solvant lipophile généralement utilisé dans l'industrie des arômes, tel qu'un solvant alcoolique, le méthanol ou l'éthanol, un solvant chloré, le chlorure de méthylène ou le dichloréthane, ou un hydrocarbure aromatique ou aliphatique, comme le benzène ou l'hexane, en général à la température de reflux du solvant; on peut éventuellement utiliser des mélanges de solvant ou faire deux extractions successives avec des solvants différents. Il est préférable d'utiliser des solvants alcooliques ou chlorés, qui sont de meilleurs solvants des composés terpéniques que les hydrocarbures, et mieux le méthanol et le chlorure de méthylène. On peut aussi, pour éliminer les composés hydrosolubles entraînés lors de cette extraction, redissoudre le résidu d'évaporation dans un solvant non miscible à l'eau, comme le chlorure de méthylène et laver la phase organique à l'eau, avant d'éliminer de nouveau le solvant organique.

La seconde étape d'oxydation enzymatique est effectuée dans des conditions classiques, dans l'eau, éventuellement en présence d'un agent tensioactif comme un ester de sorbitol et d'acide gras (Tween®) ou d'un tiers solvant améliorant la dispersibilité dans le milieu aqueux des molécules hydrophobes, sans dénaturer les enzymes, comme le dioxanne, le tétrahydrofuranne ou un alcool, à la température et au pH les plus favorables à l'activité de l'enzyme, en général entre 25°C et 35°C et à un pH alcalin.

On trouve des lipoxygènases dans de nombreux végétaux : l'aubergine, la tomate, la pomme de terre ou le soja; celle généralement utilisée qui est commerciale est la lipoxygènase de soja. Les peroxydases sont aussi présentes dans de nombreux végétaux, mais la plus courante est celle de radis noir; on peut aussi citer les manganèses peroxydases et les chloroperoxydases, isolables de différents micro-organismes ou la lactoperoxydase du lait.

On sait que l'irone formée est susceptible d'être dégradée par oxydation et la durée de la réaction ne sera pas prolongée au-delà du temps nécessaire à sa formation. Le spécialiste sera à même de déterminer, par des essais préalables, les concentrations des réactifs dans le milieu et la durée de la réaction les plus favorables; l'évolution de la réaction peut être suivie en chromatographie en phase gazeuse.

L'irone sera isolée par entraînement à la vapeur ou par extraction dans un solvant non miscible à l'eau.

Dans ce qui suit, on décrit des exemples de mise en oeuvre de l'invention.

### EXEMPLE 1

### 1ère étape : extraction des précurseurs

2,5 kg de rhizomes d'iris Pallida d'Italie, séchés mais contenant encore 60% d'eau, sont nettoyés et mis dans un récipient en inox de 60 l comprenant un bloc rotatif central à lames avec 24 l d'éthanol à 96%; le mélange est maintenu 20 h à la température de reflux du solvant, puis ramené à température ambiante et filtré; le filtrat est concentré sous vide à siccité; les solides séparés sont remis dans le récipient avec 12 l de chlorure de méthylène et le mélange est maintenu pendant 20 h à la température de reflux du solvant, puis filtré à température ambiante, le filtrat est concentré sous vide à siccité et le résidu, réuni au précédent, est redissous dans 2 l de chlorure de méthylène. La phase organique est lavée deux fois à l'eau et évaporée à siccité pour donner 1 kg d'extrait sec, qui sera soumis à la peroxydation enzymatique.

### 2ème étape : obtention d'irone

10 g d'extrait sont mis en suspension dans 90 ml d'une solution aqueuse tamponnée avec du borate de sodium (0,01 M, pH 9,2); on ajoute 10 ml de dioxanne et 0,17 g de lipoxygènase de soja à 6 unités par mg, commercialisée par Fluka. Le mélange est maintenu, sous forte agitation, en atmosphère d'oxygène à 30°C pendant 24 h; on effectue alors un entraînement à la vapeur du milieu réactionnel, par environ 1 l d'eau; l'irone entraînée est dosée par chromatographie en phase gazeuse. On obtient ainsi 0,77 g d'irone par kg de poids sec de rhizome de départ.

Lorsque la réaction est prolongée durant 48 h, on obtient 1,8 g d'irone par kg de poids sec de rhizome; l'oxydation chimique donne sensiblement le même résultat.

### EXEMPLE 2

On applique le même mode opératoire qu'à l'exemple 1, mais on introduit, lors de la 2ème étape dans le milieu réactionnel, de l'acide linolèique à la concentration 10⁻³M.

On obtient alors, après 24 h, 1,8 g d'irone et, si la réaction est prolongée jusqu'à 48 h, 2,2 g d'irone par kg de poids sec de rhizome de départ.

### EXEMPLE 3

10 g d'extrait en solution dans 20 ml de dioxanne sont introduits dans 100 ml de tampon phosphate (pH 8, 10 mM) avec 40 mg de peroxydase de raifort, et 0,3 »mole d'eau oxygénée. La peroxydase a une activité enzymatique de 100 unités/mg, une unité correspondant à 1 »mole de gaïacol transformé par min et par mg d'enzyme. Après 60 h, l'irone formée est entraînée à la vapeur. On isole ainsi 680 mg d'irone par kg de poids sec de rhizome; sans enzyme, il ne se forme que 50 mg/kg d'irone.

### EXEMPLE 4

On applique le même mode opératoire qu'à l'exemple 3, mais on introduit aussi dans le milieu 4,38 mg d'acide ascorbique. Il se forme alors en 24 h 400 mg/kg d'irone et en 60 h 450 mg/kg.

## Revendications

1. Procédé d'obtention d'irone, caractérisé en ce qu'on soumet les précurseurs extraits des rhizomes d'iris frais à l'action d'un système enzymatique peroxydant comprenant de l'oxygène et une lipoxygènase ou de l'eau oxygénée et une peroxydase.

2. Procédé selon la revendication 1, caractérisé en ce que l'enzyme est une lipoxygènase.

3. Procédé selon la revendication 2, caractérisé en ce que le milieu réactionnel contient, en outre, un acide carboxylique aliphatique insaturé susceptible de donner un hydroperoxyde en présence de lipoxygènase.

4. Procédé selon la revendication 3, caractérisé en ce que le milieu réactionnel contient de l'acide linolénique ou de l'acide linoléique.

5. Procédé selon la revendication 1, caractérisé en ce que l'enzyme est une peroxydase.

6. Procédé selon l'une quelconque des revendications précédentes, caractérisé en ce qu'on introduit dans le milieu réactionnel un agent tensioactif ou un tiers solvant.

7. Procédé selon la revendication 6, caractérisé en ce que le solvant est choisi parmi le dioxanne et le tétrahydrofuranne.

8. Procédé selon l'une quelconque des revendications précédentes, caractérisé en ce que les précurseurs sont extraits des rhizomes par un solvant lipophile choisi parmi les solvants alcooliques, les solvants chlorés, les hydrocarbures aliphatiques et aromatiques et leurs mélanges.

9. Procédé selon la revendication 8, caractérisé en ce que le solvant est choisi parmi le méthanol, l'éthanol et le chlorure de méthylène.

## Claims

1. Process for obtaining irone, characterized in that the precursors extracted from fresh iris rhizomes are subjected to the action of a peroxidizing enzymatic composition comprising oxygen and a lipoxidase or hydrogen peroxide and a peroxidase.

2. Process according to claim 1, characterized in that the enzyme is a lipoxidase.

3. Process according to claim 2, characterized in that the reaction medium further contains an unsaturated aliphatic carboxylic acid capable of giving rise to a hydroperoxide in the presence of a lipoxidase.

4. Process according to claim 3, characterized in that the reaction medium further contains linolenic acid or linoleic acid.

5. Process according to claim 1, characterized in that the enzyme is a peroxidase.

6. Process according to any one of the preceding claims, characterized in that a surfactant or a co-solvent is introduced into the reaction medium.

7. Process according to claim 6, characterized in that the solvent is selected from dioxanne and tetrahydrofuranne.

8. Process according to any one of the preceding claims, characterized in that the precursors are extracted from rhizomes by a lipophilic solvent selected from alcoholic solvents, chlorinated solvents, aliphatic and aromatic hydrocarbons and their mixtures.

9. Process according to claim 8, characterized in that the solvent is selected from methanol, ethanol or methylene chloride.

## Patentansprüche

1. Verfahren zur Herstellung von Ironen, dadurch gekennzeichnet, daß die aus frischen Iris-Wurzelstöcken extrahierten Vorläuferverbindungen der Einwirkung eines peroxidierenden enzymatischen Systems unterzogen werden, das Sauerstoff und eine Lipoxygenase oder Wasserstoffperoxid und eine Peroxidase enthält.

2. Verfahren nach Anspruch 1, dadurch gekennzeichnet, daß das Enzym eine Lipoxygenase ist.

3. Verfahren nach Anspruch 2, dadurch gekennzeichnet, daß das Reaktionsmedium außerdem eine ungesättigte aliphatische Carbonsäure enthält, die in Gegenwart der Lipoxygenase in ein Hydroperoxid übergeführt werden kann.

4. Verfahren nach Anspruch 3, dadurch gekennzeichnet, daß das Reaktionsmedium Linolensäure oder Linolsäure enthält.

5. Verfahren nach Anspruch 1, dadurch gekennzeichnet, daß das Enzym eine Peroxidase ist.

6. Verfahren nach einem der vorhergehenden Ansprüche, dadurch gekennzeichnet, daß dem Reaktionsmedium ein grenzflächenaktiver Stoff oder ein weiteres Lösungsmittel zugegeben wird.

7. Verfahren nach Anspruch 6, dadurch gekennzeichnet, daß das Lösungsmittel unter Dioxan und Tetrahydrofuran ausgewählt ist.

8. Verfahren nach einem der vorhergehenden Ansprüche, dadurch gekennzeichnet, daß die Vorläuferverbindungen mit einem lipophilen Lösungsmittel, das unter alkoholischen Lösungsmitteln, chlorierten Lösungsmitteln, aliphatischen und aromatischen Kohlenwasserstoffen sowie Gemischen dieser Lösungsmittel ausgewählt ist, aus Wurzelstöcken extrahiert werden.

9. Verfahran nach Anspruch 8, dadurch gekennzeichnet, daß das Lösungsmittel unter Methanol, Ethanol und Methylenchlorid ausgewählt ist.
